# EUROPEAN PATENT APPLICATION

(11) **EP 3 915 462 A1**
(43) Date of publication of application: **01.12.2021**
(21) Application number: 20177113.6
(22) Date of filing: 28.05.2020
(51) Int. Cl.: A61B 5/00, A61B 5/04

(54) **ELECTRODE DEVICE, ELECTROPHYSIOLOGICAL RECORDING SYSTEM AND COMPUTER PROGRAM**

(71) Applicant: Leibniz-Institut für Neurobiologie Magdeburg, 39118 Magdeburg (DE)
(72) Inventor: TAKAGAKI, Kentaroh, 39104 Magdeburg (DE); LIPPERT, Michael, 39104 Magdeburg (DE); XIA, Zifeng, 39104 Magdeburg (DE); HERRERA-MOLINA, Rodrigo, 39104 Magdeburg (DE)
(74) Representative: Gramm, Lins & Partner Patent- und Rechtsanwälte PartGmbB

(57) **Abstract**

The invention is related to an electrode device (1) for recording electrophysiological neurosignals (N1, N2, N3) in nervous tissue (13) of a living being, comprising a bundle of insulated electrical cables (2), each cable (2) having an electrical wire (3) made of electrically conductive material and an insulation layer (4) which covers and thereby insulates the electrical wire (3), the electrode device (1) further comprises an electrical connector (8) for connecting the electrical wires (3) to a recording device (10) and a free end (5) of the bundle distant from the electrical connector (8), wherein the bundle comprises an implantation section (7) at or close to its free end (5), the implantation section (7) being designated for implantation in the nervous tissue (13) of the living being. The invention is further related to an electrophysiological recording system comprising at least one such electrode device (1) and to a computer program arranged for execution on a computer (11) of such an electrophysiological recording system.

## Description

The invention is related to an electrode device for recording electrophysiological neurosignals in nervous tissue of a living being, comprising a bundle of insulated electrical cables, each cable having an electrical wire made of electrically conductive material and an insulation layer which covers and thereby insulates the electrical wire, the electrode device further comprises an electrical connector for connecting the electrical wires to a recording device and a free end of the bundle distant from the electrical connector, wherein the bundle comprises an implantation section at or close to its free end, the implantation section being designated for implantation in the nervous tissue of the living being. The invention is further related to an electrophysiological recording system comprising at least one such electrode device and to a computer program arranged for execution on a computer of such an electrophysiological recording system.

Currently, there is an increasing need in clinical translational medicine and in basic physiology research, to record from as many locations as possible electrical signals in electrically active living tissue, in particular, in the brain or other nervous tissue. In present electrode devices this is achieved by simply increasing the number of electrical cables used for recording the electrophysiological neurosignals. Microcircuit integration and miniaturization of such cables and circuitry have allowed recording of many locations, for example, in the new Neuropixel probes, which allow recording from hundreds of electrical cables.

Despite miniaturization there is a physical limit to the number of recording channels which can be included in such a recording probe when one follows the established approach of having one electrical cable per recording location. In modern multi-recording probes, the size is significantly increased, which leads to significant tissue trauma and damage. This hinders the use in chronic applications and for widespread human treatment and diagnostics.

It is therefore an object of the invention to provide for an electrode device, an electrophysiological recording system and a computer program which overcomes the drawbacks of the prior art, in particular the physical limits due to the increased size.

The present invention solves this problem by an electrode device for recording electrophysiological neurosignals in nervous tissue of a living being, comprising a bundle of insulated electrical cables, each cable having an electrical wire made of electrically conductive material and an insulation layer which covers and thereby insulates the electrical wire, the electrode device further comprises an electrical connector for connecting the electrical wires to a recording device and a free end of the bundle distant from the electrical connector, wherein the bundle comprises an implantation section at or close to its free end, the implantation section being designated for implantation in the nervous tissue of the living being, wherein one, several or all of the electrical cables have at least one side opening in the insulation layer distant from the free end, the side opening providing direct galvanic contact of the electrical wire to the nervous tissue.

In the prior art proposals each electrical wire had only one galvanic contact point which is the cut free end of the electrical cable where the wire is exposed. This principle allows only for one galvanic contact point per electrical cable.

Different to the prior art, where only the free end of a cable allows for a direct galvanic contact of the electrical wire to the nervous tissue, the present invention proposes the aforementioned side openings in the insulation layer of the electrical cables. Such side opening can also be called a lateral opening in the insulation layer.

In this way, the electrical wire can be connected through this side opening to the nervous tissue. This provides the basis for a completely novel form of measurement and recording of electrophysiological neurosignals in a way that can be called "source multiplexing" principle. Since the electrical wires have a certain longitudinal extension along the implantation section, there is a possibility to provide more than one side opening in the insulation layer of a single electrical cable within the implantation section. As a result, one single electrical cable could be used for electrically contacting the nervous tissue at different longitudinal positions along the electrical wire within the implantation section. When several of these electrical cables are prepared with several side openings, which are arranged in a certain geometrical pattern, the electrical signals which can be measured at the electrical connector of the electrode device comprise an overlapping (superposition) of the electrophysiological neurosignals. However, with the knowledge of the geometrical pattern of the side openings it is possible, using a demultiplexing algorithm, to select and distinguish the original electrophysiological neurosignals out of the electrical singles measured at the electrical connector of the electrode device.

This completely novel form of analog amplitude source multiplexing allows each electrical cable in the bundle to carry information from a large number of geometric locations in the nervous tissue. Namely, different combinatoric subsets of the electrical wires of the electrode device can be exposed in a geometrically controlled manner at specific locations in the nervous tissue, in order to pick up electrical signals from excitable cells at multiple recording locations in the nervous tissue. By passing the electrical signals to a defined unique subset of combinations of electrical wires, instead of a signal from one location to a labelled-line single electrical wire, by the present invention it is possible to multiplex signals from a large number of recording locations onto a small number of electrical wires, or to be more precise, onto a large number of wire combinations carried out by a small number of electrical wires.

As a result, with the present invention excitable cells such as neurons at a location A can be sensed by a specific combination of electrical cables, whereas neurons at a different location B can be sensed by a different combination of cables. Therefore, at least some of the electrical cables of the electrode device of the present invention can carry information from more than one location in the nervous tissue.

With an increasing number of electrical wires, the potential number of combinatoric combinations rises dramatically, resulting in, for example, around 255 different sensing locations for an electrode device having 8 electrical cables, minus some unpractical combinations, featuring for example combinations of electrical wires which do not approach each other geometrically within the target region of the nervous tissue.

Technically, a combinatoric subset recording is achieved in the electrical wires by introducing desheathing openings in the side insulation of the electrical wires, arranged so that different combinations of electrical wires have side openings at specific locations.

Furthermore, it is well known within the field of electrophysiology that recording from a single location leads to vulnerability towards artifact and movement of the recording tip, since the precise electrical waveform in this one channel is the only evidence that one is recording from the same excitable cell (e.g. neuron). The use of multiple wires allows the differentiation of unique neurons, based on the distribution of their action potential waveform shapes ("spikes") across the various wires (so-called "tetrode" effect). The precursor of this type of electrode is called a microwire multitrode (stereotrode, triode, tetrode, heptrode, etc.), and is one of the most common tools for in viva neuronal recordings in the mammalian brain. The downside of this precursor electrode is that multiple wires are needed to record from one location in the brain, making the recording per wire/lead yield even worse than the standard electrodes described in the previous paragraph.

The electrode of the present invention overcomes also these drawbacks of the prior art. Since electrical spikes in excitable cells are discrete events with precise timing matched at the microsecond level between multiple sensing locations, the location origin of each spike can be decomposed by an algorithm, as long as spikes do not overlap excessively. Even with substantial spike overlap, linear subtraction and/or convolution algorithms can be used to calculate the location origin to a large extent. Furthermore, depending on the spike firing rate of the investigated area, different numbers of side openings per electrical cable can be applied for avoiding such signal saturation.

Such design of the electrical wires ensures pick up of the electrical signals in the nervous tissue including also electrical spikes, by all co-localized side openings where the side openings are close or even abutting each other and the side openings have good impedance characteristics to attain a sufficient signal-to-noise ratio, despite the multiple tissue contacts per electrical wire. According to an advantageous embodiment of the invention, the electrical wires can be processed with impedance reduction features, such as electrochemical roughening or deposition of nanochemical layers of molecular conductors, ionic polymers or metals.

The electrical cables can be, for example, conventional electric cables with a single wire or a litz wire, or can be solid-state leads. The electrical cables can each have their own insulation layer. It is also possible that two or more or all cables comprise an insulation layer which is made in one piece among the cables. For example, the bundle of insulated electrical cables can be made by a solid-state manufacturing process or by a 3D printing process.

The side openings in the insulation layer can be produced directly or simultaneously in such manufacturing process, or they can be produced later by producing an opening in the already completed insulation layer of a cable. For example, the side openings can be produced by mechanical methods, like cutting processes, or by optical or laser ablation, ultrasound machining, electron or ion-beam etching, electrical discharge machining, heat ablation, chemical ablation or micromechanical machining.

The electrical wire of an electrical cable can be a relatively thin wire, for example a wire with a diameter in the range of 5 to 500 µm, or more preferably in the range of 10 to 100 µm. The insulation layer can have a thickness in the range of 1 to 50 µm. For example, the material for the insulation layer can be a polymer.

The tip of the electrical wire can be electrically insulated. This allows for sharpening or other geometric modifications of the free end of an electrical cable which might otherwise interfere with electrode contact site design.

The electrical connector for connecting the electrical wires to the recording device can be located, for example, on one end of the bundle, in particular on the end of the bundle which is distant from the free end.

According to an advantageous embodiment of the invention, one, several or all electrical cables have more than one side opening in the insulation layer. Each side opening provides for a desheathing area in the insulation layer, which allows direct galvanic contact of the electrical wire to the nervous tissue. By such plurality of side openings in each of the one, several or all electrical cables the number of sensing locations in the nervous tissue can be significantly increased.

According to an advantageous embodiment of the invention, the side openings in the insulation layer of a cable are distant from each other in the longitudinal direction of the cable. In other words, there is at least some portion of the insulating material of the insulating layer between neighboring side openings of a cable.

The longitudinal direction is the direction in which the cable has its largest dimension (length).

According to an advantageous embodiment of the invention, one, several or all electrical cables have insulation material of the insulation layer at their free end, which covers and thereby isolates the free end of the electrical wire. This allows for sharpening or other geometric modifications at the tip of the electric cable.

According to an advantageous embodiment of the invention, at least some of the side openings on the different electrical cables are at different longitudinal positions. This allows for recording of electrophysiological neurosignals at different longitudinal positions of the electrode device and therefore at different depths in the nervous tissue.

According to an advantageous embodiment of the invention, the bundle comprises at least one pair of electrical cables having at least one of their side openings at the same longitudinal position. In this way, the aforementioned demultiplexing of the signals arriving at the electrical connector of the electrode device can be supported. For example, the bundle can comprise several pairs of electrical cables which each have at least one of their side openings at the same longitudinal position. It is also possible that there are more than two electrical cables, namely not only a pair but maybe a triple and so on, having at least one of their side openings at the same longitudinal position. This allows for improved demultiplexing of the signals at the electrical connector, in particular in case of a large number of electrical cables in the bundle.

According to an advantageous embodiment of the invention, the electrical cables are in a wound, twisted and/or woven structure thereby establishing the bundle of electrical cables. This allows for easy handling of the electrode device and safe implantation within the nervous tissue. In case an electrode device with a large number of electrical wires and resulting in a large diameter of the bundle with complex geometry, weaving processes such as the traditional Japanese kumihimo patterns can be used to ensure that each wire combination comes in close special proximity at some point along the electrode device at which the side openings for this combination of cables would be placed.

The electrode device with its electrical cables can also be manufactured as a thin-film polymer, silicon waver process, or 3D printing process, thereby bringing the different combinations of electrical wires close enough together along the electrode to guarantee combinatoric pick up of spikes from sufficient number of neurons at each side by the electric wire at the side openings in the insulation layer in each location.

According to an advantageous embodiment of the invention, the side openings of the electrical cables of the bundle establish a geometrical pattern which allows to record electrophysiological neurosignals at different positions in the nervous tissue, whereby the geometrical pattern allows for distinguishing the electrophysiological neurosignals from each other by applying a demultiplexing algorithm to the electrical signals measured at the electrical connector of the electrode device. This rule allows for easy manufacture of the electrode device. The electrode device is of a high quality such that the electrophysiological neurosignals can be distinguished from each other by applying the demultiplexing algorithm to the electrical signals measured at the electrical connector of the electrode device. As a result, the original electrophysiological neurosignals in the nervous tissue can be reproduced and recorded, for example by a recording device of an electrophysiological recording system, for example a system as mentioned hereinafter.

The object of the invention is further achieved by an electrophysiological recording system, comprising at least one electrode device of the aforementioned kind and a recording device connected to the electrical connector of the electrode device, wherein the recording device comprises a demultiplexing algorithm for demultiplexing the electrical signals measured at the electrical connector of the electrode device and for outputting the demultiplexed and thereby distinguished signals as the electrophysiological neurosignals of the living being at different positions in the nervous tissue. With such an electrophysiological recording system the same advantages can be achieved as mentioned before. For example, the recording device may comprise a computer which executes a computer program which comprises the demultiplexing algorithm.

The object of the invention is further achieved by a computer program arranged for execution on a computer of an electrophysiological recording system of the aforementioned kind, wherein the computer program comprises the demultiplexing algorithm. With such a computer program the same advantages can be achieved as mentioned before.

The computer can be any commercially available computer, like a PC, Laptop, Notebook, Tablet or Smartphone, or a microprocessor, microcontroller or FPGA, or a combination of such elements.

In an advantageous embodiment of the invention, the demultiplexing algorithm is arranged for calculating the location origin of the electrophysiological neurosignals in the nervous tissue from the electrical signals measured at the electrical connector of the electrode device. This allows for precise reproduction of the original electrophysiological neurosignals and its assignment to the correct location origin of the electrophysiological neurosignals in the nervous tissue. In an advantageous embodiment of the invention, the demultiplexing algorithm can comprise a linear subtraction and/or a convolution algorithm for calculating the location origin of the electrophysiological neurosignals in the nervous tissue.

The invention is further described by exemplary embodiments which are depicted in the drawings. The drawings shown in
- Figure 1: an electrophysiological recording system and
- Figure 2: the process of signal demultiplexing in the system of figure 1.

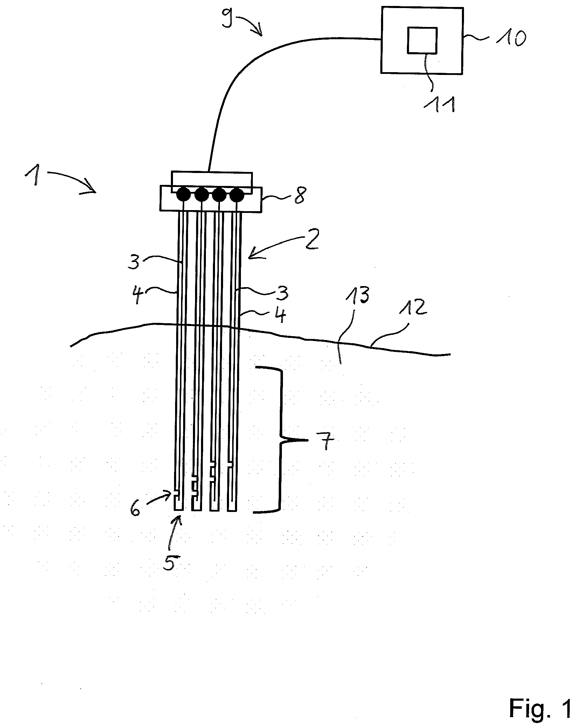

The exemplary embodiment of figure 1 depicts an electrode device 1 which is connected by a cable 9 to a recording device 10. The electrode device 1 and the recording device 10 form an electrophysiological recording system for recording electrophysiological neurosignals in nervous tissue 13 of a living being.

For example, the electrode device 1 can be implanted with its implantation section 7 within a part 12 of the living being, for example the brain. The implantation section 7 is located at some place within the nervous tissue 13.

The electrode device 1 comprises a plurality of electrically insulated cables 2 which extend in a longitudinal direction from an electrical connector 8 to a free end 5. The cables 2 form a bundle of insulated electrical cables which ends at the free end 5. The electrical connector 8 can be a usual electrical plug-in connector. It can comprise standard termination techniques and plug assemblies.

Each electrical cable 2 has an electrical wire 3 made of electrically conductive material. Further, each cable 2 has an insulation layer 4 which covers the electrical wire 3 of the respective cable 2. In this way, the electric wire 3 of a cable 2 is insulated against the ambience.

The electrical wires 3 of the cables 2 are also covered by the insulating material of the insulating layer 4 at the free end 5. In order to provide direct galvanic contact of the electrical wire 3 to the nervous tissue 13, there are one or more side openings 6 in each cable 2 within the implantation section 7. For example, the leftmost cable 2 has only one side opening 6 which is, compared to the other cables 2, at the closest position to the free end 5. The next cable 2, right of the leftmost cable 2, has two side openings 6. One of these side openings 6 is at the same longitudinal position as the side opening 6 of the leftmost cable 2, and the other side opening 6 is more distant from the free end 5.

The next cable 2 to the right has also two side openings 6. One of the side openings 6 is at the same longitudinal position as the upper side opening of the second cable 2. The second side opening 6 of the third cable 2 is more distant from the free end 5. The fourth cable 2, which means the rightmost cable 2, has only one side opening 6 at the same longitudinal position as the upper side opening of the neighboring cable 2.

While the cables 2 are shown for simplicity in a parallel manner, in an actual practical realization the cables 2 would be twisted, wound and/or woven into a bundle.

The connection cable 9 is connected via a plug-in connector to the electrical connector 8 of the electrode device 1. Via the connection cable 9 the electrical signals which are coupled through the side openings 6 into the electrical wires 3 are fed into the recording device 10. The recording device 10 comprises signal receiving and evaluation circuitry. In particular, the recording device 10 comprises a computer 11 which executes a computer program. The computer program can comprise signal evaluation algorithms, like a demultiplexing algorithm for demultiplexing the electrical signals measured at the electrical connector 8.

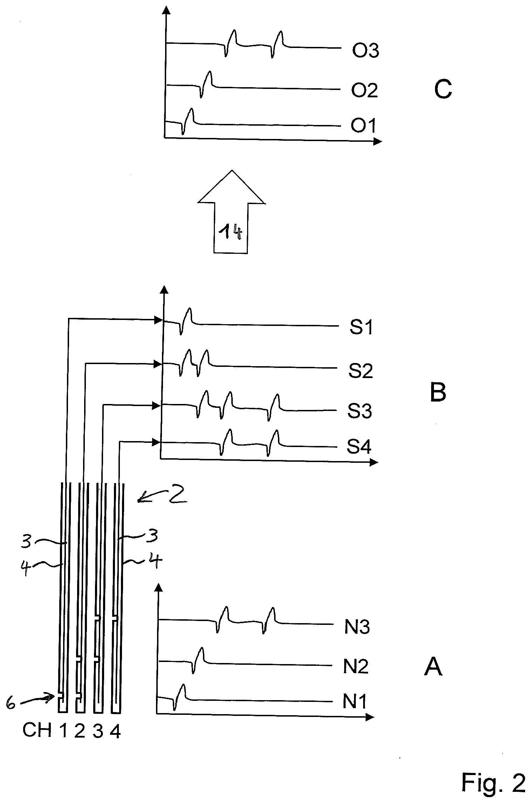

Figure 2 shows an example of the measuring of the electrophysiological signals in the nervous tissue 13 and their transfer to the recording device 10 and the demultiplexing within the recording device 10. Diagram A shows the electrophysiological signals N1, N2, N3 appearing at different locations within the nervous tissue 13. The signals N1, N2, N3 can be voltage signals over time. These signals N1, N2, N3 are sensed by the electrical wires 3 at their side openings 6. It should be clear that in a practical application not only one signal per side opening 6 is measured, but a series of such signals over time (e.g. N1a, N1b, N1c, ..., N2a, N2b, N2c, ..., N3a, N3b, N3c, ...).

Diagram B shows the electrical signals S1, S2, S3, S4 appearing on the electrical connector 8 of the electrode device 1. Signal S1 is the same as signal N1, because the leftmost cable 2 (CH1) has only one side opening 6. Signal S2 is a superposition of the signals N1 and N2, due to the two side openings 6 in the second cable 2 (CH2). Signal S3 is a superposition of the signals N2 and N3, due to the two side openings 6 in the third cable 2 (CH3). Signal S4 is the same as signal N3, because the rightmost cable 2 (CH4) has only one side opening 6.

These signals S1, S2, S3, S4 are transferred to the recording device 10 and processed by the demultiplexing algorithm 14. The result of the demultiplexing algorithm 14 is shown in diagram C. Diagram C comprises signals O1, O2, O3, which are the same as signals N1, N2, N3. In particular, signal 01 is the reproduction of signal N1, signal O2 is the reproduction of signal N2 and signal O3 is the reproduction of signal N3.

## Claims

1. Electrode device (1) for recording electrophysiological neurosignals (N1, N2, N3) in nervous tissue (13) of a living being, comprising a bundle of insulated electrical cables (2), each cable (2) having an electrical wire (3) made of electrically conductive material and an insulation layer (4) which covers and thereby insulates the electrical wire (3), the electrode device (1) further comprises an electrical connector (8) for connecting the electrical wires (3) to a recording device (10) and a free end (5) of the bundle distant from the electrical connector (8), wherein the bundle comprises an implantation section (7) at or close to its free end (5), the implantation section (7) being designated for implantation in the nervous tissue (13) of the living being, **characterized in that** one, several or all of the electrical cables (2) have at least one side opening (6) in the insulation layer (4) distant from the free end (5), the side opening (6) providing direct galvanic contact of the electrical wire (3) to the nervous tissue (13).

2. Electrode device according to claim 1, **characterized in that** one, several or all electrical cables (2) have more than one side opening (6) in the insulation layer (4).

3. Electrode device according to claim 2, **characterized in that** the side openings (6) in the insulation layer (4) of a cable (2) are distant from each other in the longitudinal direction of the cable (2).

4. Electrode device according to any of the preceding claims, **characterized in that** one, several or all electrical cables (2) have insulation material of the insulation layer (4) at their free end (5), which covers and thereby isolates the free end (5) of the electrical wire (3).

5. Electrode device according to any of the preceding claims, **characterized in that** at least some of the side openings (6) on the different electrical cables (2) are at different longitudinal positions.

6. Electrode device according to any of the preceding claims, **characterized in that** the bundle comprises at least one pair of electrical cables (2) having at least one of their side openings (6) at the same longitudinal position.

7. Electrode device according to any of the preceding claims, **characterized in that** the electrical cables (2) are in a wound, twisted and/or woven structure thereby establishing the bundle of electrical cables (2).

8. Electrode device according to any of the preceding claims, **characterized in that** the side openings (6) of the electrical cables (2) of the bundle establish a geometrical pattern which allows to record electrophysiological neurosignals (N1, N2, N3) at different positions in the nervous tissue (13), whereby the geometrical pattern allows for distinguishing the electrophysiological neurosignals (N1, N2, N3) from each other by applying a demultiplexing algorithm to the electrical signals (S1, S2, S3, S4) measured at the electrical connector (8) of the electrode device (1).

9. Electrophysiological recording system, comprising at least one electrode device (1) according to any of the preceding claims and a recording device (10) connected to the electrical connector (8) of the electrode device (1), wherein the recording device (10) comprises a demultiplexing algorithm (14) for demultiplexing the electrical signals (S1, S2, S3, S4) measured at the electrical connector (8) of the electrode device (1) and for outputting the demultiplexed and thereby distinguished signals (O1, O2, O3) as the electrophysiological neurosignals of the living being at different positions in the nervous tissue (13).

10. Electrophysiological recording system according to claim 8, **characterized in that** the demultiplexing algorithm (14) is arranged for calculating the location origin of the electrophysiological neurosignals (N1, N2, N3) in the nervous tissue (13) from the electrical signals (S1, S2, S3, S4) measured at the electrical connector (8) of the electrode device (1).

11. Computer program arranged for execution on a computer (11) of an electrophysiological recording system according to any of the claims 8 to 9, comprising the demultiplexing algorithm (14).
